# FASCICULE DE BREVET EUROPEEN

(11) **EP 1 681 040 B1**
(45) Date de publication et mention de la délivrance du brevet: **24.12.2008**
(21) Numéro de dépôt: 06290001.4
(22) Date de dépôt: 03.01.2006
(51) Int. Cl.: A61F 2/92

(54) **Endoprothèse pour canal anatomique**
Endoprothese für ein Körperlumen
Endoprosthesis for an anatomical lumen

(30) Priorité: 18.01.2005 FR 0500500
(43) Date de publication de la demande: 19.07.2006
(73) Titulaire: Novatech SA, 13600 La Ciotat (FR)
(72) Inventeur: Ferreyrol, Bruno, 13830 Roquefort-La-Bedoule (FR)
(74) Mandataire: Bonnetat, Christian

(56) Documents cités:
- EP-A- 1 393 766
- US-A- 5 236 446
- US-A1- 2002 082 679
- US-B1- 6 254 632
- US-B1- 6 506 437
- US-B1- 6 641 607

## Description

La présente invention concerne une endoprothèse qui est destinée à être implantée à l'intérieur d'un canal anatomique, dans une zone de celui-ci subissant une altération et dont la surface extérieure de la paroi porte des tétons saillants destinés à coopérer avec la paroi dudit canal anatomique pour y maintenir ladite endoprothèse en position.

Une telle endoprothèse est décrite dans le document US-5 236 446 qui représente l'état de la technique le plus proche.

Une telle altération peut être une affection, telle qu'une sténose ou un collapsus, entraînant un rétrécissement dudit canal anatomique et combattu, de façon connue, à l'aide d'une endoprothèse implantée lorsque la réduction du diamètre dudit canal est importante, par exemple supérieure à 50%. Cette endoprothèse maintient ouvert le passage dudit canal au niveau de ladite zone, ce qui permet la circulation des fluides à travers celle-ci. L'action d'une telle endoprothèse est donc essentiellement un tuteurage mécanique.

On sait de plus que l'implantation d'une telle endoprothèse de maintien d'ouverture est généralement associée à un traitement médical adapté, tel qu'administration de médicaments anti-inflammatoires, radiothérapie, chimiothérapie, etc ... Or, ce traitement médical est appliqué de façon générale au patient, c'est-à-dire par voie anale, orale ou par perfusion pour les médicaments et les produits chimiques et par irradiation à travers le corps du patient pour la radiothérapie. Il en résulte que les doses appliquées au patient sont très supérieures aux doses qui se retrouvent au niveau de la zone d'implantation de la prothèse, de sorte qu'il en résulte l'une ou l'autre des situations suivantes :
- soit on ne veut pas appliquer au patient un traitement général trop violent, et alors les doses du traitement au niveau de ladite zone d'implantation sont très inférieures à ce qu'elles devraient être pour combattre efficacement ledit rétrécissement ;
- soit on veut que les doses au niveau de la zone d'implantation soient médicalement efficaces, et alors le patient est soumis à des doses générales très élevées, pouvant nuire à sa santé.

Outre la sténose et le collapsus rappelés ci-dessus, un canal anatomique peut subir d'autres altérations, telles que des lésions, des infections, des inflammations, des irritations, des hypersensibilités, etc ... Ces dernières altérations, qui jusqu'à présent n'entraînent pas l'usage d'une endoprothèse, nécessitent un traitement médical qui, comme mentionné ci-dessus à propos de la sténose et du collapsus, est appliqué de façon générale au patient, avec les mêmes inconvénients.

Aussi, la présente invention a pour objet de remédier à ces inconvénients, quelle que soit l'altération du canal anatomique.

A cette fin, selon l'invention, l'endoprothèse pour canal anatomique, qui est destinée à être implantée dans une zone de celui-ci subissant une altération et dont la surface extérieure de la paroi porte des tétons saillants destinés à coopérer avec la paroi dudit canal anatomique pour y maintenir ladite endoprothèse en position, est remarquable en ce qu'au moins l'un desdits tétons saillants est creux et sert de réservoir apte à contenir un produit actif contre ladite altération et à permettre l'action dudit produit actif contre cette dernière.

Ainsi, ladite endoprothèse délivre ledit produit actif à l'emplacement même de la zone altérée du canal anatomique. Ce produit actif peut donc être dosé de façon adaptée et appropriée au traitement à effectuer sur cette zone.

L'endoprothèse conforme à la présente invention constitue donc un moyen de traitement, principal ou complémentaire, de l'altération du canal anatomique. Dans le cas où, jusqu'à présent, l'altération (lésion, infection, inflammation, irritation, hypersensibilité, ...) ne relevait pas de l'implantation d'une endoprothèse, l'endoprothèse selon l'invention a pour objet spécifique de délivrer le produit actif in situ. En revanche, dans le cas où l'altération (sténose, collapsus) nécessite un tuteurage mécanique, l'endoprothèse selon l'invention peut résulter d'une endoprothèse déjà existante perfectionnée pour y incorporer au moins un réservoir de produit actif : l'endoprothèse ainsi perfectionnée permet donc, à la fois, le tuteurage du canal anatomique et la délivrance du produit actif.

La présente invention concerne donc, d'une part, des endoprothèses totalement nouvelles aptes au traitement d'altérations pour lesquelles aucune endoprothèse n'avait encore été utilisée, et, d'autre part, des endoprothèses connues, perfectionnées pour permettre la délivrance in situ d'au moins un produit actif.

L'endoprothèse conforme à la présente invention peut, après épuisement dudit produit actif, être rechargée par voie endoscopique ou être remplacée par une autre convenablement chargée en produit actif.

Dans le cas où ledit produit actif est un isotope radioactif, chaque téton creux contenant ledit produit radioactif peut être obturé par un bouchon étanche et former un réservoir étanche audit produit, dont seul le rayonnement radioactif passe en direction de ladite altération. Au contraire, chaque téton creux contenant ledit produit radioactif peut être obturé par un bouchon présentant une perméabilité pour ledit produit radioactif et permettant une diffusion contrôlée de ce dernier en direction de ladite altération.

Si le produit actif est chimique ou pharmaceutique, chaque téton creux contenant ledit produit actif est obturé par un bouchon présentant une perméabilité contrôlée pour ledit produit actif et permettant une diffusion contrôlée de celui-ci en direction de ladite altération.

En plus du ou des tétons creux servant de réservoir de produit actif, il est avantageux que l'endoprothèse selon l'invention comporte au moins un autre téton creux pour contenir un produit radio-opaque, apte à servir à la localisation de ladite endoprothèse à l'intérieur dudit canal anatomique.

Une telle endoprothèse peut, en outre, comporter, en plus des tétons creux pour servir de réservoir de produit actif et pour contenir un produit radio-opaque, au moins un autre téton contenant une pastille, par exemple d'or pur, servant de marqueur.

Dans le cas particulier où lesdits tétons de l'endoprothèse forment des lignes -par exemple longitudinales et réparties autour de ladite endoprothèse avec les tétons de deux lignes adjacentes disposés en quinconce- il est avantageux que, dans une telle ligne, un téton contienne ladite pastille servant de marqueur, alors que tous les autres tétons de ladite ligne longitudinale contiennent ledit produit radio-opaque.

Les figures du dessin annexé feront bien comprendre comment l'invention peut être réalisée. Sur ces figures, des références identiques désignent des éléments semblables.

La figure 1 illustre, en coupe axiale longitudinale, un exemple de réalisation de l'endoprothèse conforme à la présente invention.

La figure 2 est une vue de dessus de l'endoprothèses de la figure 1.

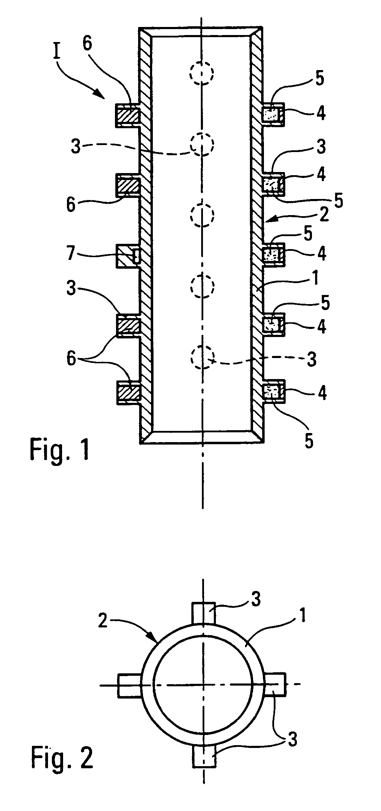

L'endoprothèse tubulaire I, conforme à la présente invention et montrée sur les figures 1 et 2, peut être réalisée en toute matière souple, semi-rigide ou rigide, renforcée ou non par une armature interne, apte à être bien tolérée par l'organisme du patient dans lequel elle doit être implantée. Toutefois, de préférence, elle est réalisée en un élastomère à base de silicone présentant de bonnes propriétés de déformation élastique.

A des fins de clarté, l'endoprothèse tubulaire I représentée est formée d'une portion de tube rectiligne et est apte à former une endoprothèse trachéale ou bronchique. Toutefois, il est bien évident que la présente invention concerne également des endoprothèses tubulaires de formes plus compliquées, par exemple courbes, en diabolo ou en Y, comme par exemple cela est nécessaire pour les endoprothèses trachéo-bronchiques. De façon générale, l'endoprothèse conforme à la présente invention est adaptée, en forme et en diamètre aux canaux à l'intérieur desquels elle doit être implantée.

L'endoprothèse I, montrée à titre d'exemple par les figures 1 et 2, est du type décrit par le document US-5 236 446. Elle comporte un corps tubulaire 1 dont la surface extérieure de sa paroi 2 porte une pluralité de tétons 3, répartis en lignes longitudinales et espacés les uns des autres dans le sens longitudinal et dans le sens périphérique et destinés à coopérer avec la paroi du canal d'implantation pour maintenir ladite endoprothèse en position. Les extrémités du corps tubulaire 1 sont ouvertes et biseautées intérieurement.

Conformément à la présente invention, au moins certains des tétons 3 sont creux et sont obturés par des bouchons 4. Ainsi, chacun de ces tétons creux 3 peut servir de réservoir apte à contenir un produit 5 actif contre l'altération, à l'emplacement de laquelle l'endoprothèse I sera implantée. Dans le cas où le produit actif 5 est un isotope radioactif agissant par irradiation, les bouchons 4 des tétons 3 correspondants sont soit poreux pour permettre une diffusion contrôlée du produit, soit étanches pour ne laisser passer que le rayonnement radioactif. Dans le cas où le produit actif 5 est un médicament ou une matière chimique, les bouchons 4 des tétons 3 correspondants sont du type membrane osmotique et présentent une perméabilité maîtrisée, adaptée aux caractéristiques de ce médicament ou de cette matière chimique, telle que taille de leur molécule, viscosité, etc ...

Par ailleurs, d'autres tétons creux 3 sont emplis d'une matière radio-opaque 6, par exemple un silicone chargé de sulfate de baryum, et servent à localiser l'endoprothèse I dans le canal d'implantation à l'aide de rayons X.

De plus, un téton 3, creux ou plein, peut contenir une pastille d'or pur 7 servant de signature à l'endoprothèse I.

Dans l'exemple de la figure 1, une ligne longitudinale complète de tétons 3 porte la matière opaque 6, à l'exclusion du téton central 3 contenant la pastille d'or pur 7.

## Revendications

1. Endoprothèse pour canal anatomique, qui est destinée à être implantée dans une zone de celui-ci subissant une altération et dont la surface extérieure de la paroi (2) porte des tétons saillants (3) destinés à coopérer avec la paroi dudit canal anatomique pour y maintenir ladite endoprothèse en position,
**caractérisée en ce qu'**au moins l'un desdits tétons saillants (3) est plein et contient un matériau radio-opaque (7) servant de marqueur et **en ce qu'**au moins un autre desdits tétons saillants (3) est creux et sert de réservoir apte à contenir un produit (5) actif contre ladite altération et à permettre l'action dudit produit actif (5) contre cette dernière.

2. Endoprothèse selon la revendication 1, apte à être mise en oeuvre avec un produit actif (5) de type radioactif,
**caractérisée en ce que** chaque téton creux (3) contenant ledit produit radioactif (5) est obturé par un bouchon étanche (4) et forme un réservoir étanche audit produit, dont seul le rayonnement radioactif passe en direction de ladite altération.

3. Endoprothèse selon la revendication 1, apte à être mise en oeuvre avec un produit actif (5) de type radioactif,
**caractérisée en ce que** chaque téton creux (3) contenant ledit produit radioactif (5) est obturé par un bouchon (4) présentant une perméabilité pour ledit produit radioactif (5) et permettant une diffusion contrôlée de ce dernier en direction de ladite altération.

4. Endoprothèse selon la revendication 1, apte à être mise en oeuvre avec un produit actif, de type chimique ou pharmaceutique,
**caractérisée en ce que** chaque téton creux (3) contenant ledit produit actif (5) est obturé par un bouchon (4) présentant une perméabilité contrôlée pour ledit produit actif et permettant une diffusion contrôlée de celui-ci en direction de ladite altération.

5. Endoprothèse selon l'une quelconque des revendications 1 à 4,
**caractérisée en ce que,** en plus du ou des tétons creux (3) servant de réservoir de produit actif, ladite endoprothèse comporte au moins un autre téton creux (3) pour contenir un produit radio-opaque (6).

6. Endoprothèse selon la revendication 5, dans laquelle lesdits tétons (3) destinés à coopérer avec la paroi du canal d'implantation forment des lignes longitudinales,
**caractérisée en ce que,** dans une telle ligne, un téton plein contient ledit matériau radio-opaque (7) servant de marqueur, alors que tous les autres tétons (3) de ladite ligne sont creux et contiennent ledit produit radio-opaque (6).

## Claims

1. An endoprosthesis for an anatomical canal, which is intended to be implanted in a region of this canal that is experiencing impairment and of which the exterior surface of the wall (2) bears projecting studs (3) intended to collaborate with the wall of said anatomical canal in order to hold said endoprosthesis in position therein,
**characterized in that** at least one of said projecting studs (3) is solid and contains a radiopaque material (7) acting as a marker and **in that** at least one of said projecting studs is hollow and acts as a reservoir able to contain a product (5) that is active against said impairment and to allow said active product (5) to act against this impairment.

2. The endoprosthesis as claimed in claim 1 able to be used with an active product (5) of the radioactive type,
**characterized in that** each hollow stud (3) containing said radioactive product (5) is plugged by a sealed stopper (4) and forms a reservoir sealed against said product, only the radioactive radiation of which passes toward said impairment.

3. The endoprosthesis as claimed in claim 1 able to be used with an active product (5) of radioactive type,
**characterized in that** each hollow stud (3) containing said radioactive product (5) is plugged by a stopper (4) exhibiting permeability to said radioactive product (5) and allowing the latter to permeate in a controlled manner toward said impairment.

4. The endoprosthesis as claimed in claim 1 able to be used with an active product of chemical or pharmaceutical type,
**characterized in that** each hollow stud (3) containing said active product (5) is plugged by a stopper (4) exhibiting controlled permeability to said active product and allowing this product to permeate in a controlled manner toward said impairment.

5. The endoprosthesis as claimed in one of claims 1 to 4,
**characterized in that,** in addition to the hollow stud or studs (3) serving as a reservoir of active product, said endoprosthesis comprises at least one other hollow stud (3) to contain a radiopaque product (6).

6. The endoprosthesis as claimed in claim 5, in which said studs (3) intended to collaborate with the wall of the implantation canal form longitudinal lines,
**characterized in that,** in such a line, one solid stud contains said radiopaque material (7) acting as a marker while all the other studs of said line are hollow and contain said radiopaque product (6).

## Patentansprüche

1. Endoprothese für einen anatomischen Kanal, die dazu bestimmt ist, in einen Bereich des Letztgenannten implantiert zu werden, der von einer Veränderung betroffen ist, wobei die Außenfläche ihrer Wand (2) vorspringende Ansätze (3) trägt, die dazu bestimmt sind, mit der Wand des anatomischen Kanals zusammenzuarbeiten, um hier die Endoprothese in Position zu halten,
**dadurch gekennzeichnet, dass** mindestens einer der vorspringenden Ansätze (3) voll ist und ein für radioaktive Strahlung undurchlässiges Material (7) enthält, das als Marker dient, und dass mindestens ein anderer der vorspringenden Ansätze (3) hohl ist und als Behälter dient, der geeignet ist, ein Produkt (5) zu enthalten, das gegenüber der Veränderung aktiv ist, und die Einwirkung des aktiven Produkts (5) gegenüber der Letztgenannten zu ermöglichen.

2. Endoprothese nach Anspruch 1, die geeignet ist, mit einem aktiven Produkt (5) des radioaktiven Typs eingesetzt zu werden,
**dadurch gekennzeichnet, dass** jeder hohle Ansatz (3), der das radioaktive Produkt (5) enthält, durch einen dichten Pfropfen (4) verschlossen wird und einen gegenüber dem Produkt dichten Behälter bildet, aus dem nur die radioaktive Strahlung in Richtung auf die Veränderung entweicht.

3. Endoprothese nach Anspruch 1, die geeignet ist, mit einem aktiven Produkt (5) des radioaktiven Typs eingesetzt zu werden,
**dadurch gekennzeichnet, dass** jeder hohle Ansatz (3), der das radioaktive Produkt (5) enthält, durch einen Pfropfen (4) verschlossen wird, der eine Durchlässigkeit gegenüber dem radioaktiven Produkt (5) aufweist und eine kontrollierte Verteilung des Letztgenannten in Richtung auf die Veränderung ermöglicht.

4. Endoprothese nach Anspruch 1, die geeignet ist, mit einem aktiven Produkt des chemischen oder pharmazeutischen Typs eingesetzt zu werden,
**dadurch gekennzeichnet, dass** jeder hohle Ansatz (3), der das aktive Produkt (5) enthält, durch einen Pfropfen (4) verschlossen wird, der eine kontrollierte Durchlässigkeit gegenüber dem aktiven Produkt aufweist und eine kontrollierte Verteilung des Letztgenannten in Richtung auf die Veränderung ermöglicht.

5. Endoprothese nach einem der Ansprüche 1 bis 4,
**dadurch gekennzeichnet, dass** die Endoprothese zusätzlich zu dem oder den hohlen Ansätzen (3), die als Behälter für das aktive Produkt dienen, mindestens einen anderen hohlen Ansatz (3) umfasst, um ein für radioaktive Strahlung undurchlässiges Produkt (6) zu enthalten.

6. Endoprothese nach Anspruch 5, wobei die hohlen Ansätze (3), die dazu bestimmt sind, mit der Wand des Implantationskanals zusammenzuarbeiten, longitudinale Linien bilden, **dadurch gekennzeichnet, dass** in einer solchen Linie ein voller Ansatz das für radioaktive Strahlung undurchlässige Material (7) enthält, das als Marker dient, während alle anderen Ansätze (3) der Linie hohl sind und das für radioaktive Strahlung undurchlässige Produkt (6) enthalten.
